# EUROPEAN PATENT SPECIFICATION

(11) **EP 3 331 358 B1**
(45) Date of publication and mention of the grant of the patent: **17.02.2021**
(21) Application number: 16751537.8
(22) Date of filing: 03.08.2016
(51) Int. Cl.: A01N 25/04, A01N 25/08, C12N 11/14, C12M 1/40

(54) **PEST PROTECTIVE APPARATUS FOR CONTROLLING PLANT PATHOGENS**
PFLANZENSCHUTZVORRICHTUNG ZUR BEKÄMPFUNG VON PHYTOPATHOGENEN
DISPOSITIF DE PROTECTION DES PLANTES POUR CONTRÔLER DES PHYTOPATHOGÈNES

(30) Priority: 07.08.2015 LU 92794
(43) Date of publication of application: 13.06.2018
(73) Proprietor: Luxembourg Institute of Science and Technology (LIST), 4362 Esch-sur-Alzette (LU)
(72) Inventor: PRINTZ, Bruno, 57330 Escherange (FR)
(74) Representative: Lecomte & Partners
(86) International application number: PCT/EP2016/068512
(87) International publication number: WO 2017/025402

(56) References cited:
- WO-A1-94/23573
- WO-A1-2014/205550
- WO-A1-2015/013808
- DE-A1- 3 312 214
- US-A- 5 163 247
- DATABASE WPI Week 197820 Thomson Scientific, London, GB; AN 1978-35958A XP002744693, -& JP S53 38627 A (ASAHI CHEM IND CO LTD) 8 April 1978 (1978-04-08)
- DATABASE WPI Week 199012 Thomson Scientific, London, GB; AN 1990-088317 XP002744694, -& JP H02 42921 A (HANNAKA T) 13 February 1990 (1990-02-13)
- DATABASE WPI Week 199629 Thomson Scientific, London, GB; AN 1996-283367 XP002744695, -& JP H08 119812 A (SUMITOMO CHEM CO LTD) 14 May 1996 (1996-05-14)
- EMRE BASTÜRK ET AL: "Covalent immobilization of [alpha]-amylase onto thermally crosslinked electrospun PVA/PAA nanofibrous hybrid membranes", JOURNAL OF APPLIED POLYMER SCIENCE, vol. 127, no. 1, 4 May 2012 (2012-05-04), pages 349-355, XP055040389, ISSN: 0021-8995, DOI: 10.1002/app.37901
- SHIWEN WANG ET AL: "Mesosilica-coated ultrafine fibers for highly efficient laccase encapsulation", NANOSCALE, vol. 6, no. 12, 1 January 2014 (2014-01-01), page 6468, XP055214038, ISSN: 2040-3364, DOI: 10.1039/c4nr01166j

## Description

### Technical field

The invention is directed to a pest protective apparatus comprising a system which allows the transformation of a substrate into a biopesticide and/or repellent product which will subsequently control plant pathogens.

### Background art

Pest management is currently largely performed with synthetic chemicals, but the development of alternative and eco-friendly methods to control pests are encouraged.

Patent application published US 2012/0079625 A1 relates to a method for protecting living plants from harmful insects via a sheet-like structure impregnated with an insecticide which is from synthetic origin.

The field of the biocontrol includes various techniques of integrated pest management such as macro-organisms, micro-organisms, pheromones, kairomones and natural substances originating from animals, minerals and plants to control pest and pathogens. In 2014, Sahayaraj K. summarized the current use of the nanotechnologies in the domain of the plant protection. The few data available on this topic brings to the conclusion that only few research all over the world are currently working with this emerging problematic although there is an urgent need of modern approaches of pest management (Sahayaraj K., Adv. Plant Biopest. (chapter 14), 2014, 279-293).

Substances from various plant families have been reported to exert a repelling activity against fungus, bacteria, nematodes or insects and can be successfully used in the treatment/control of pests (Murthy N.B.K., et al., Indian J. Exp. Biol., 1974, 12, 208-209 and Regnault-Roger C., et. al., Biopesticides d'origine végétale (2ème edition), 2008**.)** In particular, sulphur containing molecules such as those produced among other by the families *Brassicaceae* and members of the genus *Allium* which are enzymatically produced from non-toxic precursors are toxic at low level for a wide range of organisms (Ahuja I., et al., Agron. Sustain. Dev., 2010, 30, 311-348; Auger J., et al., Ecologie, 1994, 93-101; Cutler H.G., et al., Biologically Active Natural Products: Agrochemicals, 1999**).**

The mixing of biopesticide precursor with active enzymes in liquid form to produce repellent is known to the art and has been proposed for the production of glucosinolate products such as nitrile, thiocyanate or isothiocyanate, and/or mixture (WO 2015/013808 A1). Also known in the art is the use of a two-part pesticide precursor system comprising a glucosinolate concentrate on one side and an active myrosinase complex on the other side both in dry form. When both components are mixed with water, the glucosinolate breakdown products are released to control pests (US 2015/0005172 A1). However, the lifespan of active enzymes in the environment is relatively low, which considerably hinders the reusability of the enzymes. In addition, the substrates are commonly hydrophilic, making them easily leachable and not accessible to the enzymes.

Enhancing the lifespan of enzymes has been successfully performed in the domains of the biocatalysis, biofuels, enzyme-controlled drug delivery and the biosensors by immobilizing the enzymes into silica mesoporous material (Carlsson N., et al., Adv. Colloid Interface Sci., 2013, 205, 339-360; Popat A., et al., Nanoscale, 2011, 3, 2801-2818; Wang Y., et al., Chem. Mater., 2005, 17, 953-961). Mesoporous material relates to silica-based material with a pore diameter sufficient to allow the penetration of the enzyme. It can be in the form of SBA-15 (Santa Barbara-15), SBA-16, MCM-41 (Mobil Crystalline Materials-41) or all other type of mesoporous silica sieves/spheres/cages (FSM-16 (Folded Sheet Mesoporous-16), MCM-48, FDU-12 (Fudan University-12), MCF (Mesostructured Cellular Foam), SMS (Sponge Mesoporous Silica), mesoporous carbon, PMOs (Periodic Mesoporous Organosilica), Meso-MOFs (meso-Metal Organic Frameworks) (Zhou Z., et al., Top Catal., 2012, 55, 1081-1100). The pore structure may be folded-sheet, 2D hexagonal channels, cubic, spherical cages, mesocellular foam, sponge-like mesoporous silica, 3D cubic cages. The synthesis of the mesoporous silica is preferentially done in acidic conditions and with the triblock copolymer (Pluronic®P123) as template and tetraethyl orthosilicate (TEOS) as silica source. However, the use of other templates such as among other the triblock copolymer (Pluronic®F127), CTMA (cetyltrimethylammonium) (CₙH₂ₙ₊₁(CH₃)N⁺, n=8-18), CTAB (cetyltrimethylammonium bromide) and other silica source including among other (3-aminopropyl)trimethoxysilane (APTMOS), sodium silicate or tetramethyl orthosilicate (TMOS) may also give interesting properties to the silica mesoporous material (Zhou Z., et al., Top Catal., 2012, 55, 1081-1100).

Enzyme immobilization into the silica matrix can be carried out through various techniques including cross-linking methods, covalent binding, physical adsorption, encapsulation and entrapment (CN 101451133 B). Carboxylethyl or aminopropyl functionalization of the mesoporous silica also appears as a good technique for improving the catalytic activities of the silica-based biocomposite (Lei C., et al., J. Am. Chem. Soc., 2002, 124, 11242-11243). Functionalization of the silica-material can further be used for covalent linking of the enzyme on the derivatized-silica.

The synthesis of hydrophilic gels can result from the gelation of various natural compounds such as agarose, agar-agar, alginate, pectin, starch or gelatine taken alone or in mixes. They allow the diffusion of small hydrophilic molecules and prevent the diffusion of large particles such as silica mesoporous materials that retain blocked in the polymeric network. Microencapsulation of silica in alginate had remarkable advantages of sustained-release of compounds and stability under different pH values, different temperatures, and UV irradiation. The presence of the hydrophilic gel can further increase the stability of the material upon ageing and limit enzyme leaching (Coradin T., et al., Comptes Rendus Chim., 2003, 6, 147-152). Biocomposite incorporation in hydrophilic gels such as alginate showed much better performance due to the more homogeneous distribution of silica particles in the composite material (Xu S. et al., Ind. Eng. Chem. Res., 2006, 45, 511-517).

The document Database WPI Week 197820, AN 1978-35958A discloses a pest protective apparatus for controlling weeds comprising a fibrous network characterised in that said fibrous network comprises a system which releases a herbicide

The document Database WPI Week 199012, AN 1990-088317 discloses a pest protective apparatus for controlling plant pathogenic fungi comprising a fibrous network.

US 5 163 247 A discloses a pest protective apparatus for controlling plant pathogens comprising a fibrous network coated with a latex further including antimicriobial agent, a pesticide or a fungicide.

WO94/23573 A1 discloses a network comprising a polymeric fibre wherein an agriculturally active ingredient has been uniformly dispersed.

The document Database WPI Week 199629, AN 1996-283367 discloses a pest protective apparatus for controlling plant pathogenic insects comprising a fibrous network characterised in that said fibrous network comprises a system which releases an insecticide.

E. Bastürk et al. (Journal of Applied Polymer Sciences, vol. 127, n°1, p. 349-355, 2012) discloses a fibrous network made from thermally crosslinked electrospun polyvinyl alcohol/polyacrylic acid.

Shiwen Wang et al. (Nanoscale, vol. 6, n°12, p. 6468, 2014) discloses a fibre comprising a coating with mesoporous silica and the enzyme laccase (an oxidase) absorbed therein.

DE 33 12 214 A1 discloses immobilised myrosinase (a thioglucosidase). Especially preferred for the immobilisation of myrosinase are acidic inorganic ion exchangers, such as zeolites or aluminosilicates.

WO2014/205550 A1 discloses a method for providing a pesticide, said method consisting in the provision of a two-part precursor system comprising a in a first part a glucosinolate concentrate and in a second part an active myrosinase complex in a plant material, wherein the composition will release a biopesticide when both parts are mixed with water

The idea of the present invention is to merge all these parts (conception of a structure, using a precursor with active enzymes in liquid form to produce biopesticide and/or repellent product, enhancing the lifespan of said enzymes, use of hydrophilic gels) to produce for the first time a pest protective apparatus which is able to deliver *in situ* a constant flux of biopesticide and/or repellent product.

### Summary of invention

### Technical Problem

The invention has for technical problem to provide a pest protective apparatus which is producing a biopesticide and/or repellent product from a precursor which is harmless and easy to handle.

### Technical solution

The first object of the present invention is a pest protective apparatus, for controlling plant pathogens, comprising at least one fibrous network and/or at least one fibrous cover according to claim 1. Preferred embodiments are recited in the following paragraphs and in dependent claims.

In one embodiment, said at least one active enzyme is immobilized within one nanoporous material.

In one embodiment, said one nanoporous material is a mesoporous material

In one embodiment, said nanoporous material is embedded within one hydrophilic gel.

In one embodiment, said hydrophilic gel is made of agarose, agar-agar, alginate, pectin, starch and/or gelatine, preferentially made of alginate.

In one embodiment, said at least one active enzyme is one active enzyme selected from the group of glycosidase.

In one embodiment, said at least one enzyme from the group of glycosidase is thioglucosidase.

In one embodiment, said at least one active enzyme immobilized within said nanoporous material is covalently bound to said nanoporous material, physically adsorbed to said nanoporous material, encapsulated within said nanoporous material or entrapped within said nanoporous material.

In one embodiment, said at least one active enzyme immobilized within said nanoporous material is cross-linked with glutaraldehyde or with 1-ethyl-3-(3-dimethylaminopropyl)carbodiimide and *N*-hydroxysuccinimide.

In one embodiment, said nanoporous material is a silica-based mesoporous material.

In one embodiment, said silica-based mesoporous material comprises tetraethyl orthosilicate and/or tetramethyl orthosilicate and/or methyltrimethoxysilane and/or (3-aminopropyl)trimethoxysilane.

In one embodiment, said preparation has a pH comprised between 4 and 11.

In one embodiment, said preparation further comprises at least one mineral and/or at least one cofactor.

In one embodiment, said preparation is adapted to receive said at least one precursor, at least one mineral and/or at least one cofactor.

In one embodiment, said at least one mineral and/or said at least one cofactor is phosphate buffer or ascorbic acid.

In one embodiment, said phosphate buffer is at a concentration comprised between 10 mM and 500 mM, preferentially at a concentration comprised between 50 mM and 250 mM, more preferentially at a concentration comprised between 75 mM and 150 mM, even more preferentially at a concentration of 100 mM.

In one embodiment, said ascorbic acid is at a concentration comprised between 50 µM and 1500 µM, preferentially at a concentration comprised between 150 µM and 1000 µM, more preferentially at a concentration comprised between 250 µM and 750 µM, even more preferentially at a concentration of 500 µM.

In one embodiment, said cartridge further comprises at least one mineral and/or at least one cofactor.

It is a second object of the present invention to disclose a method for controlling plant pathogens according to claim 14 with a pest protective apparatus according to claim 1 and to dependent claims thereof The first step of activating at least one precursor may also be, for example, the catalytic splitting of said at least one precursor.

In one embodiment, said at least one precursor is comprised in the pest protective apparatus, said pest protective apparatus preferentially also comprising at least one mineral and/or at least one cofactor.

In one embodiment, said at least one precursor is added to said pest protective apparatus, said pest protective apparatus preferentially comprising at least one mineral and/or at least one cofactor.

### Advantages of the invention

The invention is particularly interesting in that the precursor of the biopesticide and/or repellent product which is provided by the pest protective apparatus of the invention is chosen among the inactive and non-toxic forms of the potential organic derivatives. The biopesticide and/or repellent preparation which is coated onto the fibres of the fibrous network and/or fibrous cover further comprises immobilized enzyme which provides enhanced enzymatic activity compared to a non-immobilized enzyme.

### Brief description of the drawings

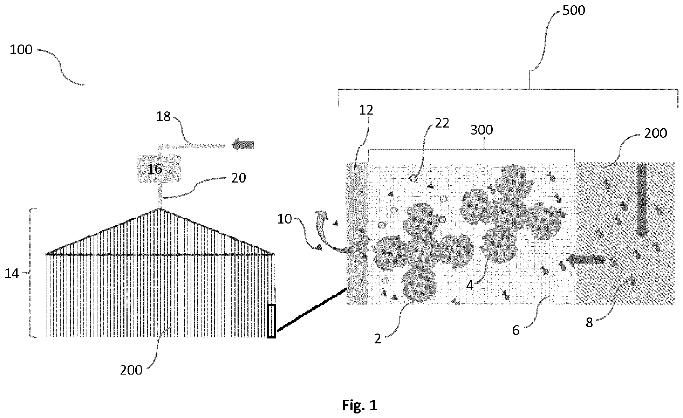

Figure 1: Pest protective apparatus.

### Description of an embodiment

The present invention relates to a pest management biocontrol system made of active biocatalysts able to produce natural repellents and/or biopesticides by means of controlled enzymatic reactions.

As depicted on figure 1, the pest protective apparatus 100 of the present invention comprises at least one fibrous network and/or at least one fibrous cover 14.

Said network and/or said cover 14 comprises a system 500 in which at least one precursor 8 is transformed into a biopesticide and/or repellent product 10.

The system 500 is made of fibres 200 which are in fact coated with a preparation 300 which provides 'biopesticide and/or repellent properties. Such type of preparation comprises at least one active enzyme 4 which is capable to transform at least one substrate 8 into a biopesticide and/or repellent product 10, which will subsequently control plant pathogens.

In order to enhance the lifespan of said at least one enzyme 4, the biocatalyst is immobilized within one nanoporous material 2, preferably into a mesoporous silica matrix, more particularly into a silica-based mesoporous material, the whole being glued or embedded into a hydrophilic gel 6.

The mesoporous materials of the present invention are ordered silica-based mesoporous particles with a narrow pore size distribution, a well-defined pore geometry and a well-defined pore connectivity.

The pore size distribution and the global geometry of the mesoporous material are defined according to the properties of the biopesticide-producing enzymes and/or repellent-producing enzymes that are immobilized into the mesoporous structure.

The pore size of the silica-based mesoporous material is comprised between 5 nm and 30 nm

The immobilization procedure should allow to optimize the catalytic activities of the enzymes in comparison with the free enzymes and to enhance their reusability in order to produce a constant and sufficient flux of biopesticide and/or repellent product allowing a sufficient biopesticide and/or repellent activity against the targeted pathogens.

The silica-based mesoporous material will be synthesized in acidic conditions using a silica precursor that could be among other tetraethyl orthosilicate (TEOS), and/or tetramethyl orthosilicate (TMOS), and/or methyltrimethoxysilane (MTMOS), and/or (3-aminopropyl)triemthoxysilane (APTMOS), in combination with tri-block copolymer mixtures such as Pluronic®F127 and/or Pluronic®F123 as structure-directing agents.

The mesoporous structure can be produced hydrothermally and/or by solgel synthesis and can be functionalized.

The enzymes that may be immobilized into the mesoporous material are those involved in the natural responses of the plants when facing a pathogen attack. The biopesticide and/or repellent compounds produced during the enzymatic reaction can be a bactericide, a fungicide, an insecticide and/or a nematicide.

The enzyme from the glycosidases, carbon-sulfur lyases and/or lachrymatory-factor synthases can be immobilized into the mesoporous material.

Preferred glycosidases are the enzymes hydrolysing O-glycosyl and S-glycosyl compounds.

Preferred are also carbon-sulfur lyases, in particular alliin lyase (also known as alliinase).

Enzymes from the PF10604 family, i.e. the lachrymatory-factor synthase, can also be used in the present invention.

A preferred glycosidase is thioglucosidase. This is the enzyme of choice which has been tested for transforming a precursor into a biopesticide and/or repellent product.

The immobilization procedure can be performed through the following techniques known in the art: covalent binding, physical adsorption, encapsulation and entrapment.

The immobilization includes the penetration of the enzymes inside the mesoporous material, through the nanopores and an eventual step of cross-linkage between the enzymes using gluturaldehyde or 1-ethyl-3-(3-dimethylaminopropyl)carbodiimide and *N*-hydroxysuccinimide.

The mesoporous biocatalyst will then be embedded in a hydrophilic gel 6 made of agarose, agar-agar, alginate, pectin, starch, gelatine taken alone or in mixes, preferentially alginate.

The use of alginate allows a gelation without heating. Only CaCl₂ must be added for the gelation step.

The hydrophilic gel allows the diffusion of the products of the enzymatic reaction, the diffusion of the substrates of the enzymes and the confinement of all chemicals required to maintain the enzymes in a working status.

The hydrophilic gel 6 may contain all minerals and cofactors indispensable for the enzymatic activities. This can be a phosphate buffer (pH = 6.1) at a concentration comprised between 10 mM and 500 mM, preferentially at a concentration of 100 mM and/or ascorbic acid at a concentration comprised between 50 µM and 1500 µM, preferentially at a concentration of 500 µM.

In case the minerals and cofactors are not added before the gelation step, they can be added by diffusion within the gel by soaking into an aqueous buffer comprising these compounds.

The pH of preparation 300 able to produce a biopesticide and/or a repellent for controlling plant pathogens is preferably comprised between 4 and 11. In particular, the pH of the hydrophilic gel allows an optimal activity of the immobilized enzymes.

Upon addition of the substrate 8 for the enzyme 4 and upon addition of water, and, upon addition of minerals and cofactors for activating the enzyme if they are not formerly included within said hydrophilic gel 6, the enzyme 4 will proceed to the synthesis of a product 10 which will present biopesticide and/or repellent properties. This product 10 will be released and will act to eliminate (in case of biopesticide activity) and/or to keep away (in case of repellent activity) the pest and/or the plant pathogens from the living plants, such as bacteria, fungi, insects, bugs, and/or nematodes.

The scheme on figure 1 represents in particular a precursor 8 composed of two chemical moieties, one being the biopesticide and/or the repellent product 10, the other being a compound 22.

Preferentially, the product 10 of the enzymatic reaction is a volatile organic compound.

The fibres 200 of the pest protective apparatus 100, which are coated with the preparation 300 able to produce biopesticide and/or repellent for controlling plant pathogens, are preferably natural fibres. Such natural fibres might be for example hemp, flax, nettle, cotton, jute, ramie, sisal, and/or any other.

Preferentially, the fibres are hemp or flax.

The substrate 8 of the enzyme 4 is able to diffuse within the fibre, in order to reach the preparation 300 able to produce biopesticide and/or repellent for controlling plant pathogens where said substrate 8 will be processed by the active enzyme 4.

Those fibres 200 are further coated by a protective layer, which is a hydrophobic layer 12 in order to be protected from drying.

Such hydrophobic layer is also configured to be permeable to the biopesticide and/or repellent product 10.

The hydrophobic layer 12 may be (3-aminopropyl)triethoxysilane, (3-mercaptopropyl)triethoxysilane, succinic anhydride, alkylketene dimer, 3-isopropenyl-α-α-dimethylbenzyl isocyanate, m-phenylene bismaleimide, vinyl trialkoxysilane, 3-metacryloyloxy propyl trimetoxysilane and/or any other.

The moiety 22, resulting from the cleavage of the precursor 8, stays therefore within the preparation 300 able to produce biopesticide and/or repellent and does not therefore pollute the environment.

As depicted on figure 1, the pest protective apparatus 100 of the present invention may further comprise means for distributing water to said at least one fibrous network and/or said at least one fibrous cover 14, said means preferably comprising a cartridge 16. Said cartridge 16 is configured for being in fluid connection with said at least one fibrous network and/or said at least one fibrous cover 14 and for being fed with water. Said cartridge 16 may comprise at least one inlet 18 and at least one outlet 20.

Depending whether the pest protective apparatus 100 comprises or does not comprise the cartridge 16, said apparatus 100 is configured either to transform at least one precursor 8, which is incorporated to the preparation 300 able to produce biopesticide and/or repellent by diffusion through the fibre 200, into a biopesticide and/or a repellent product 10; or to incorporate said at least one precursor 8 into said cartridge 16.

Depending whether the pest protective apparatus 100 comprises or does not comprise the cartridge 16, the at least one mineral and/or the at least one cofactor indispensable for the enzyme activity are incorporated either directly within the hydrophilic gel 6 of the preparation 300 able to produce biopesticide and/or repellent; or within the cartridge 16.

The enzymatic reaction is triggered by the addition of water, which plays the role of the solvent of the reaction, bringing subsequently the substrate 8 into contact with the fibres 200 of the preparation 300 able to produce biopesticide and/or repellent, and more particularly, into contact with the active site of the enzyme.

The second utility of water is to bring to the enzyme the at least one mineral and/or the at least one cofactor indispensable for its activity.

Water can come from the rain and/or from artificial means, such for example a drain pump, a hosepipe and/or an irrigation system, and can be channelled to contact the preparation 300 able to produce biopesticide and/or repellent for controlling plant pathogens, triggering subsequently the enzymatic reaction.

When the pest protective apparatus 100 comprises a cartridge 16, the flux of water reaches the cartridge 16 through the inlet 18 which is provided on the cartridge 16.

Once the enzymatic reaction is over, the product 10, which presents biopesticide and/or repellent properties, is released. Generally, the product 10 is a volatile organic compound.

This product 10 acts to eliminate (in case of biopesticide activity) and/or to keep away (in case of repellent activity) the pests and/or the plant pathogens from the living plants, such as bacteria, fungi, insects, bugs, and/or nematodes.

The biopesticide and/or repellent product 10 resulting from the activation of the precursor 8 by addition of water to the pest protective apparatus 100 taught in the present invention is applied to plants, preferentially vegetables and/or fruits, in order to prevent pest attacks.

## Claims

1. Pest protective apparatus (100), for controlling plant pathogens, comprising at least one fibrous network and/or at least one fibrous cover (14)
comprising a system (500),
wherein said system is composed of fibres (200) which are coated with a preparation (300), said preparation (300) comprising at least one active enzyme (4) which is selected from the group of glycosidase, carbon-sulfur lyases and/or lachrymatory-factor synthase, said enzyme (4) is capable to transform at least one biopesticide and/or repellent precursor (8) into a biopesticide and/or repellent product (10) ; said apparatus (100) further comprising means for distributing water to the at least one fibrous network and/or at least one fibrous cover (14),
wherein either the at least one precursor (8) is incorporated to the preparation (300) or wherein said means are comprising a cartridge (16) configured for being in fluid connection with said at least one fibrous network and/or at least one fibrous cover (14) and for being fed with water, said cartridge (16) is comprising said at least one precursor (8) which is transformed into said product (10) into said system (500) of said at least one fibrous network and/or at least one fibrous cover (14).

2. Pest protective apparatus (100) according to claim 1, **characterized in that** said at least one active enzyme (4) is immobilized within one nanoporous material (2).

3. Pest protective apparatus (100) according to claim 2, **characterized in that** said one nanoporous material (2) is a mesoporous material.

4. Pest protective apparatus (100) according to one of claims 2 and 3, **characterized in that** said nanoporous material (2) is embedded within one hydrophilic gel (6).

5. Pest protective apparatus (100) according to claim 4, **characterized in that** said hydrophilic gel (6) is made of agarose, agar-agar, alginate, pectin, starch and/or gelatine, preferentially made of alginate.

6. Pest protective apparatus (100) according to any one of claims 1-5, **characterized in that** said at least one active enzyme (4) is one active enzyme selected from the group of glycosidase, and is preferably thioglucosidase.

7. Pest protective apparatus (100) according to claim 6, **characterized in that** where the enzyme (4) is glycosidase, the at least one biopesticide and/or repellent precursor (8) is comprising O-glycosyle and S-glycosyle compounds.

8. Pest protective apparatus (100) according to any one of claims 2-7, **characterized in that** said at least one active enzyme (4) immobilized within said nanoporous material (2) is covalently bound to said nanoporous material (2), physically adsorbed to said nanoporous material (2), encapsulated within said nanoporous material (2) or entrapped within said nanoporous material (2), said at least one active enzyme (4) immobilized within said nanoporous material (2) is preferably cross-linked with glutaraldehyde or with 1-ethyl-3-(3-dimethylaminopropyl)carbodiimide and *N*-hydroxysuccinimide.

9. Pest protective apparatus (100) according to any one of claims 1-8, **characterized in that** said preparation (300) has a pH comprised between 4 and 11.

10. Pest protective apparatus (100) according to any one of claims 1-9, **characterized in that** said preparation (300) further comprises at least one mineral and/or at least one cofactor.

11. Pest protective apparatus (100) according to claim 10, **characterized in that** said at least one mineral and/or said at least one cofactor is phosphate buffer or ascorbic acid.

12. Pest protective apparatus (100) according to claim 11, **characterized in that** said phosphate buffer is at a concentration comprised between 10 mM and 500 mM, preferentially at a concentration comprised between 50 mM and 250 mM, more preferentially at a concentration comprised between 75 mM and 150 mM, even more preferentially at a concentration of 100 mM.

13. Pest protective apparatus (100) according to claim 11, **characterized in that** said ascorbic acid is at a concentration comprised between 50 µM and 1500 µM, preferentially at a concentration comprised between 150 µM and 1000 µM, more preferentially at a concentration comprised between 250 µM and 750 µM, even more preferentially at a concentration of 500 µM.

14. A method for controlling plant pathogens with a pest protective apparatus (100), **characterized in that** said pest protective apparatus (100) is in accordance with any one of claims 1-13;
and wherein said method comprises the following steps:
a) activating the at least one precursor (8) by addition of water to the system (500) of said at least one fibrous network and/or said at least one fibrous cover (14) to produce a biopesticide and/or repellent product (10) for controlling plant pathogens;
b) applying the biopesticide and/or repellent product resulting from step (a) to plants, preferentially vegetables and/or fruits, in order to prevent pest attacks.

15. The method for controlling plant pathogens according to claim 14, **characterized in that** said pest protective apparatus (100) is comprising at least one mineral and/or at least one cofactor.

## Patentansprüche

1. Vor Schädlingen schützende Vorrichtung (100) zum Eindämmen von Pfanzenpathogenen, mindestens ein faseriges Netz und/oder mindestens eine faserige Abdeckung (14) umfassend, das bzw. die ein System (500) umfasst,
wobei das System von Fasern (200) gebildet wird, die mit einem Präparat (300) beschichtet sind, wobei das Präparat (300) mindestens ein aktives Enzym (4) umfasst, das ausgewählt ist aus der Gruppe aus Glycosidase, Kohlenstoff-Schwefel-Lyasen und/oder Synthase des tränenreizenden Faktors, wobei das Enzym (4) in der Lage ist, mindestens einen Biopestizid- und/oder Repellentvorläufer (8) in ein Biopestizid- und/oder ein Repellentprodukt (10) umzuwandeln; wobei die Vorrichtung (100) ferner eine Einrichtung zum Verteilen von Wasser auf das mindestens eine faserige Netz und/oder die mindestens einen faserige Abdeckung (14) umfasst,
wobei entweder der mindestens eine Vorläufer (8) in das Präparat (300) aufgenommen ist oder wobei die Einrichtung eine Kartusche (16) umfasst, die dafür ausgelegt ist, mit dem mindestens einen faserigen Netz und/oder der mindestens einen faserigen Abdeckung (14) in Fluidverbindung zu stehen und mit Wasser beschickt zu werden, wobei die Kartusche (16) den mindestens einen Vorläufer (8) umfasst, der in das Produkt (10) in das System (500) des mindestens einen faserigen Netzes und/oder der mindestens einen faserigen Abdeckung (14) umgewandelt wird.

2. Vor Schädlingen schützende Vorrichtung (100) nach Anspruch 1, **dadurch gekennzeichnet, dass** das mindestens eine aktive Enzym (4) innerhalb eines nanoporösen Materials (2) immobilisiert ist.

3. Vor Schädlingen schützende Vorrichtung (100) nach Anspruch 2, **dadurch gekennzeichnet, dass** das eine nanoporöse Material (2) ein mesoporöses Material ist.

4. Vor Schädlingen schützende Vorrichtung (100) nach einem der Ansprüche 2 und 3, **dadurch gekennzeichnet, dass** das nanoporöse Material (2) in ein hydrophiles Gel (6) eingebettet ist.

5. Vor Schädlingen schützende Vorrichtung (100) nach Anspruch 4, **dadurch gekennzeichnet, dass** das hydrophile Gel (6) aus Agarose, Agar-Agar, Alginat, Pektin, Stärke und/oder Gelatine hergestellt ist, vorzugsweise aus Alginat hergestellt ist.

6. Vor Schädlingen schützende Vorrichtung (100) nach einem der Ansprüche 1-5, **dadurch gekennzeichnet, dass** das mindestens eine aktive Enzym (4) ein aktives Enzym ist, das ausgewählt ist aus der Gruppe aus Glycosidase, und vorzugsweise Thioglucosidase.

7. Vor Schädlingen schützende Vorrichtung (100) nach Anspruch 6, **dadurch gekennzeichnet, dass** dann, wenn das Enzym (4) Glycosidase ist, der mindestens eine Biopestizid- und/oder Repellentvorläufer (8) O-Glycosyl- und S-Glycosylverbindungen umfasst.

8. Vor Schädlingen schützende Vorrichtung (100) nach einem der Ansprüche 2-7, **dadurch gekennzeichnet, dass** das mindestens eine aktive Enzym (4), das innerhalb des nanoporösen Materials (2) immobilisiert ist, kovalent an das nanoporöse Material (2) gebunden ist, physikalisch an das nanoporöse Material (2) adsorbiert ist, in dem nanoporösen Material (2) eingekapselt ist oder in dem nanoporösen Material (2) eingefangen ist, wobei das mindestens eine aktive Enzym (4), das in dem nanoporösen Material (2) immobilisiert ist, vorzugsweise mit Glutaraldehyd oder mit 1-Ethyl-3-(3-dimethylaminopropyl)carbodiimid und *N*-Hydroxysuccinimid vernetzt ist.

9. Vor Schädlingen schützende Vorrichtung (100) nach einem der Ansprüche 1-8, **dadurch gekennzeichnet, dass** das Präparat (300) einen pH aufweist, der zwischen 4 und 11 liegt.

10. Vor Schädlingen schützende Vorrichtung (100) nach einem der Ansprüche 1-9, **dadurch gekennzeichnet, dass** das Präparat (300) ferner mindestens ein Mineral und/oder mindestens einen Cofaktor umfasst.

11. Vor Schädlingen schützende Vorrichtung (100) nach Anspruch 10, **dadurch gekennzeichnet, dass** das mindestens eine Mineral und/oder der mindestens eine Cofaktor Phosphatpuffer oder Ascorbinsäure ist.

12. Vor Schädlingen schützende Vorrichtung (100) nach Anspruch 11, **dadurch gekennzeichnet, dass** der Phosphatpuffer eine Konzentration, die zwischen 10 mM und 500 mM umfasst, vorzugsweise eine Konzentration, die zwischen 50 mM und 250 mM umfasst, stärker bevorzugt eine Konzentration, die zwischen 75 mM und 150 mM umfasst, noch stärker bevorzugt eine Konzentration von 100 mM aufweist.

13. Vor Schädlingen schützende Vorrichtung (100) nach Anspruch 11, **dadurch gekennzeichnet, dass** die Ascorbinsäure eine Konzentration, die zwischen 50 µM und 1500 µM umfasst, vorzugsweise eine Konzentration, die zwischen 150 µM und 1000 µM umfasst, stärker bevorzugt eine Konzentration, die zwischen 250 µM und 750 µM umfasst, noch stärker bevorzugt eine Konzentration von 500 µM aufweist.

14. Verfahren zum Eindämmen von Pflanzenpathogenen mit einer vor Schädlingen schützenden Vorrichtung (100), **dadurch gekennzeichnet, dass** die vor Schädlingen schützende Vorrichtung (100) einem der Ansprüche 1-13 entspricht;
und wobei das Verfahren die folgenden Schritte umfasst:
a) Aktivieren des mindestens einen Vorläufers (8) durch die Zugabe von Wasser zu dem System (500) des mindestens einen faserigen Netzes und/oder der mindestens einen faserigen Abdeckung (14), um ein Biopestizid- und/oder Repellentprodukt (10) zum Eindämmen von Pflanzenpathogenen zu produzieren;
b) Anwenden des Biopestizid- und/oder Repellentprodukts, das sich aus Schritt (a) ergibt, auf Pflanzen, vorzugsweise auf Gemüse und/oder Obst, um Schädlingsbefall zu verhindern.

15. Verfahren zum Eindämmen von Pflanzenpathogenen nach Anspruch 14, **dadurch gekennzeichnet, dass** die vor Schädlingen schützende Vorrichtung (100) mindestens ein Mineral und/oder mindestens einen Cofaktor umfasst.

## Revendications

1. Appareil de protection (100) contre des organismes nuisibles, destiné à la lutte contre des agents phytopathogènes, comprenant au moins un maillage fibreux et/ou au moins un revêtement fibreux (14), qui comprend un système (500) ; dans lequel ledit système se compose de fibres (200) qui sont enduites d'une préparation (300), ladite préparation (300) comprenant au moins une enzyme active (4) qui est choisie parmi le groupe constitué par des glycosidases, des lyases carbone/soufre et/ou le facteur lacrymogène synthase, ladite enzyme (4) étant capable de transformer au moins un biopesticide et/ou un précurseur d'agent répulsif (8) en un biopesticide et/ou en un produit répulsif (10) ; ledit appareil (100) comprenant en outre un moyen destiné à distribuer de l'eau audit au moins un maillage fibreux et/ou audit au moins un revêtement fibreux (14) ; dans lequel, soit ledit au moins un précurseur (8) est incorporé à la préparation (300), soit ledit moyen comprend une cartouche (16) qui est configurée pour venir se mettre en communication par fluide avec ledit au moins un maillage fibreux et/ou ledit au moins un revêtement fibreux (14) et pour être alimentée avec de l'eau, ladite cartouche (16) comprenant ledit au moins un précurseur (8) qui a été transformé pour obtenir ledit produit (10) dans ledit système (500) dudit au moins un maillage fibreux et/ou dudit au moins un revêtement fibreux (14).

2. Appareil de protection (100) contre des organismes nuisibles selon la revendication 1, **caractérisé en ce que** ladite au moins une enzyme active (4) est immobilisée au sein d'une matière nanoporeuse (2).

3. Appareil de protection (100) contre des organismes nuisibles selon la revendication 2, **caractérisé en ce que** ladite une matière nanoporeuse (2) représente une matière mésoporeuse.

4. Appareil de protection (100) contre des organismes nuisibles selon l'une quelconque des revendications 2 et 3, **caractérisé en ce que** ladite matière nanoporeuse (2) est incorporée au sein d'un gel hydrophile (6).

5. Appareil de protection (100) contre des organismes nuisibles selon la revendication 4, **caractérisé en ce que** ledit gel hydrophile (6) est constitué d'agarose, d'agar-agar, d'alginate, de pectine, d'amidon et/ou de gélatine, de manière préférentielle est constitué d'alginate.

6. Appareil de protection (100) contre des organismes nuisibles selon l'une quelconque des revendications 1 à 5, **caractérisé en ce que** ladite au moins une enzyme active représente une enzyme active qui est choisie parmi le groupe des glycosidases, et représente de préférence la thioglucosidase.

7. Appareil de protection (100) contre des organismes nuisibles selon la revendication 6, **caractérisé en ce que** l'enzyme (4) représente une glycosidase, ledit au moins un biopesticide et/ou ledit au moins un précurseur d'agent répulsif (8) comprend/comprennent des composés O-glycolsylés et S-glycolsylés.

8. Appareil de protection (100) contre des organismes nuisibles selon l'une quelconque des revendications 2 à 7, **caractérisé en ce que** ladite au moins une enzyme active (4) immobilisée au sein de ladite matière nanoporeuse (2) est liée de manière covalente à ladite matière nanoporeuse (2), est adsorbée par voie physique à ladite matière nanoporeuse (2), est encapsulée au sein de ladite matière nanoporeuse (2) ou est prise au piège au sein de ladite matière nanoporeuse (2) ; ladite au moins une enzyme active (4) immobilisée au sein de ladite matière nanoporeuse (2) étant de préférence réticulée avec du glutaraldéhyde ou avec du l-éthyl-3-(3-diméthylaminopropyl)carbodiimide et du *N*-hydroxysuccinimide.

9. Appareil de protection (100) contre des organismes nuisibles selon l'une quelconque des revendications 1 à 8, **caractérisé en ce que** ladite préparation (300) possède un pH qui est compris entre 4 et 11.

10. Appareil de protection (100) contre des organismes nuisibles selon l'une quelconque des revendications 1 à 9, **caractérisé en ce que** ladite préparation (300) comprend en outre au moins une matière minérale et/ou au moins un cofacteur.

11. Appareil de protection (100) contre des organismes nuisibles selon la revendication 10, **caractérisé en ce que** ladite au moins une matière minérale et/ou ledit au moins un cofacteur représente(nt) un tampon de phosphate ou l'acide ascorbique.

12. Appareil de protection (100) contre des organismes nuisibles selon la revendication 11, **caractérisé en ce que** ledit tampon de phosphate est présent à une concentration qui est comprise entre 10 mM et 500 mM, de manière préférentielle à une concentration qui est comprise entre 50 mM et 250 mM, de manière plus préférentielle à une concentration qui est comprise entre 75 mM et 150 mM, de manière encore plus préférentielle à une concentration de 100 mM.

13. Appareil de protection (100) contre des organismes nuisibles selon la revendication 11, **caractérisé en ce que** ledit acide ascorbique est présent à une concentration qui est comprise entre 50 µM et 1500 µM, de manière préférentielle à une concentration qui est comprise entre 150 µM et 1000 µM, de manière plus préférentielle à une concentration qui est comprise entre 250 µM et 750 µM, de manière encore plus préférentielle à une concentration de 500 µM.

14. Procédé destiné à la lutte contre des agents phytopathogènes avec un appareil (100) de protection contre des organismes nuisibles, **caractérisé en ce que** ledit appareil (100) de protection contre des organismes nuisibles est conforme à l'une quelconque des revendications 1 à 13; et dans lequel ledit procédé comprend les étapes suivantes consistant à :
a) activer ledit au moins un précurseur (8) par addition d'eau au système (500) dudit au moins un maillage fibreux et/ou dudit au moins un revêtement fibreux (14) dans le but d'obtenir un biopesticide et/ou un produit répulsif (10) pour la lutte contre des agents phytopathogènes ;
b) appliquer le biopesticide et/ou le produit répulsif que l'on obtient à l'étape (a) sur des plantes, de manière préférentielle sur des légumes et/ou sur des fruits, à des fins de prévention contre des attaques d'organismes nuisibles.

15. Procédé destiné à la lutte contre des agents phytopathogènes selon la revendication 14, **caractérisé en ce que** ledit appareil (100) de protection contre des organismes nuisibles comprend au moins une matière minérale et/ou au moins un cofacteur.
